(19) **Europäisches Patentamt**
**European Patent Office**
**Office européen des brevets**

(11) **EP 2 509 599 B1**

(12) **EUROPEAN PATENT SPECIFICATION**

(45) Date of publication and mention
of the grant of the patent:
**13.08.2014 Bulletin 2014/33**

(51) Int Cl.:
*A61K 31/445* *(2006.01)*   *A61K 31/44* *(2006.01)*
*A61K 31/416* *(2006.01)*   *A61P 35/00* *(2006.01)*
*A61K 33/24* *(2006.01)*    *A61K 31/443* *(2006.01)*
*A61K 31/506* *(2006.01)*   *A61K 31/513* *(2006.01)*
*A61K 31/517* *(2006.01)*

(21) Application number: **10836725.1**

(22) Date of filing: **10.12.2010**

(86) International application number:
**PCT/US2010/059785**

(87) International publication number:
**WO 2011/072181 (16.06.2011 Gazette 2011/24)**

(54) **Method for treating pancreatic cancer**

Verfahren zur Behandlung von Bauchspeicheldrüsenkrebs

Méthode de traitement du cancer du pancréas

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.12.2009 US 285676 P**

(43) Date of publication of application:
**17.10.2012 Bulletin 2012/42**

(73) Proprietor: **Niiki Pharma Inc.**
**Hoboken, New Jersey 07030 (US)**

(72) Inventor: **SHESHBARADARAN, Hooshmand**
**New Jersey 07030 (US)**

(74) Representative: **Vossius & Partner**
**Siebertstrasse 4**
**81675 München (DE)**

(56) References cited:
• **RIVERA F ET AL: "Treatment of advanced pancreatic cancer: From gemcitabine single agent to combinations and targeted therapy", CANCER TREATMENT REVIEWS, SAUNDERS, US, vol. 35, no. 4, 1 June 2009 (2009-06-01), pages 335-339, XP026123601, ISSN: 0305-7372, DOI: 10.1016/J.CTRV.2008.11.007 [retrieved on 2009-01-07]**

• **HARTINGER C G ET AL: "From bench to bedside - preclinical and early clinical development of the anticancer agent indazolium trans-[tetrachlorobis(1H-indazole)ruthenat e(III)] (KP1019 or FFC14A)", JOURNAL OF INORGANIC BIOCHEMISTRY, ELSEVIER INC, US, vol. 100, no. 5-6, 1 May 2006 (2006-05-01) , pages 891-904, XP027900388, ISSN: 0162-0134 [retrieved on 2006-05-01]**

• **HENKE, M.M. ET AL.: 'Pharmacokinetic study of sodium trans[tetrachlorobis(1H-indazole)-ruthenate (III)]/-indazole hydrochloride (1:1.1) (FFC14A) in patie nts with solid tumors' INT. J. CLIN. PHARMACOL. THER. vol. 47, no. 1, January 2009, pages 58 - 60, XP008160855**

• **HARTINGER, C.G. ET AL.: 'From bench to bedside-preclinical and early clinical development of the anticancer agent indazolium trans-[tetrachlorobis(IH-ind azole)ruthenate(III)] (KP1019 or FFC14A)' J. INORG. BIOCHEM. vol. 100, no. 5-6, May 2006, pages 891 - 904, XP027900388**

• **KAPITZA, S. ET AL.: 'Heterocyclic complexes of ruthenium(III) induce apoptosis in colorectal carcinoma cells' J. CANCER RES. CLIN. ONCOL. vol. 131, no. 2, February 2005, pages 101 - 110, XP019345379**

• **MENDOZA-FERRI, M.G. ET AL.: 'Transferring the concept of multinuclearity to ruthenium complexes for improvement of anticancer activity' J. MED. CHEM. vol. 52, no. 4, 26 February 2009, pages 916 - 925, XP008161358**

**Description**

The present invention generally relates to treating cancer, and particularly to treating pancreatic cancer.

Pancreatic cancer is one of the most deadly forms of cancer. In the US, over forty thousand people each year are diagnosed of pancreatic cancer, and less than 5% of those survive for more than five years after diagnosis. The low survival rate is largely attributable to the fact that most pancreatic cancers are not diagnosed until an advanced stage. Pancreatic cancer is usually asymptomatic at early stage, while the symptoms at later stage are non-specific and varied, making early diagnosis difficult.

Treatment option for pancreatic cancer has been limited. Surgery and radiation therapy can be used for early-stage pancreatic cancer, but not very effective for advanced or recurrent pancreatic cancer. Weekly intravenous administration of gemcitabine has been shown to be effective and was approved in 1998 by the US FDA for pancreatic cancer.

The result of numerous clinical trials of gemcitabine and a second therapeutic agent are detailed in Rivera et al., Cancer Treatment Reviews, 2009, p335-339

The US FDA has also approved the kinase inhibitor erlotinib for use in combination with gemcitabine for patients with advanced-stage pancreatic cancer who have not received previous chemotherapy. However, the median overall survival benefit derived from erlotinib is only less than four weeks. Moore et al., J. Clin. Oncol., 25(15):1960-6 (2007).

Indazolium trans-[tetrachlorobis(1H-indazole)ruthenate(III)] and sodium trans-[tetrachlorobis(1H-indazole)ruthenate(III)] have been shown to be effective in killing tumor cells in colon caner cell lines SW480 and HT29. Kapitza et al., J. CancerRes. Clin. Oncol., 131(2):101-10 (2005). However, it is not known whether they would be effective in treating pancreatic cancer.

The invention relates to the embodiments as defined in the claims.

It has now been discovered that the compound sodium trans-[tetrachlorobis(1H-indazole)ruthenate(III)] is especially effective in treating pancreatic cancer. It has also been surprisingly discovered that the compound sodium trans-[tetrachlorobis(1H-indazole)ruthenate(III)] is equally effective in pancreatic cell lines both sensitive and insensitive to drugs such as gemcitabine and erlotinib.

Accordingly, in a first aspect, provided herein is a method of treating pancreatic caner, which comprises treating a patient identified as having pancreatic cancer, with a therapeutically effective amount of trans-[tetrachlorobis(1H-indazole)ruthenate(III)] or a pharmaceutically acceptable salt thereof.

In a second aspect, provided herein is a method of preventing or delaying the onset of pancreatic cancer, comprising administering to a patient identified to be in need of prevention, or delaying the onset, of pancreatic cancer a prophylatically effective amount of trans-[tetrachlorobis(1H-indazole)ruthenate(III)] or a pharmaceutically acceptable salt thereof.

The present invention further provides use of trans-[tetrachlorobis(1H-indazole)ruthenate(III)] or a pharmaceutically acceptable salt thereof for the manufacture of a medicament useful for treating, preventing or delaying the onset of pancreatic cancer.

1. Use of a therapeutically effective amount of a pharmaceutically acceptable salt of trans-[tetrachlorobis(1H-indazole)ruthenate(III)] for the manufacture of a medicament for treating pancreatic cancer.

2. Use of a therapeutically effective amount of a pharmaceutically acceptable salt of trans-[tetrachlorobis(1H-indazole)ruthenate(III)] for the manufacture of a medicament for preventing or delaying the onset of pancreatic cancer. In yet another aspect, provided herein is a method of treating refractory pancreatic cancer comprising identifying a patient having refractory pancreatic cancer and treating the patient with a therapeutically effective amount of trans-[tetrachlorobis(1H-indazole)ruthenate(III)] or a pharmaceutically acceptable salt thereof In specific embodiments, the patient has a pancreatic cancer that is refractory to a treatment comprising one or more drugs selected from the group consisting of gemcitabine, erlotinib, mitomycin C and platinum agents.

3. The use according to item 1, wherein said treatment is useful for preventing or delaying the onset of, a refractory pancreatic cancer.

4. The use according to item 3, wherein said cancer is refractory to a treatment comprising one or more of gemcitabine and erlotinib.

5. The use according to item 3, wherein said cancer was previously treated with and not responsive to a treatment comprising one or more of gemcitabine and erlotinib.

6. The use according to item 3, wherein said cancer is pancreatic cancer relapsing after a previous treatment comprising one or more of gemcitabine and erlotinib.

7. The use according to any one of items 4-6, wherein said previous treatment comprises gemcitabine.

8. The use according to any one of items 4-6, wherein said previous treatment comprises erlotinib.

9. The use according to any one of items 1-8, wherein said pharmaceutically acceptable salt is sodium trans-[tetrachlorobis(1H-indazole)ruthenate(III)].

The foregoing and other advantages and features of the invention, and the manner in which the same are accomplished, will become more readily apparent upon consideration of the following detailed description of the invention taken in conjunction with the accompanying examples, which illustrate preferred and exemplary embodiments.

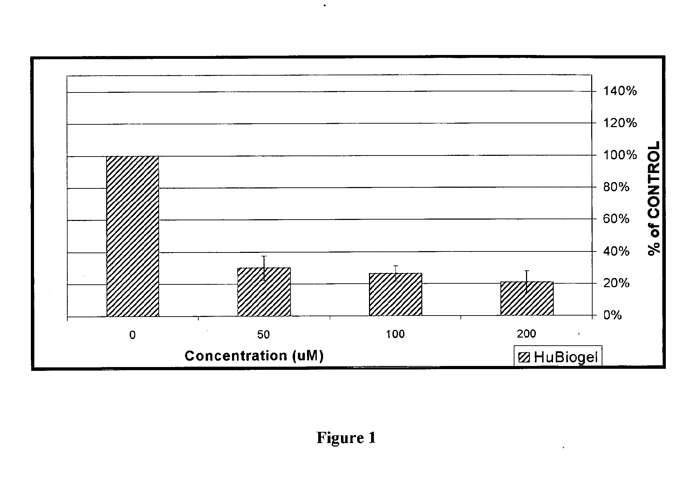

Figure 1 is a graph showing the dose-dependent growth inhibition by sodium trans-[tetrachlorobis(1H-indazole)ruthenate(III)] (MTT assay) in a 3-dimentional tumor model (HuBiogel, Vivo Biosciences, Birmingham, AL) derived from pancreatic tumor cell line MIA PaCa2.

The present invention is at least in part based on the discovery that the compound sodium trans-[tetrachlorobis(1H-indazole)ruthenate(III)] is especially effective in treating pancreatic cancer. Accordingly, in accordance with a first aspect of the present disclosure a method is provided for treating pancreatic cancer. Specifically, the method comprises treating a patient having pancreatic cancer with a therapeutically effective amount of trans-[tetrachlorobis(1H-indazole)ruthenate(III)] or a pharmaceutically acceptable salt thereof. That is, the present invention is directed to the use of trans-[tetrachlorobis(1H-indazole)ruthenate(III)] or a pharmaceutically acceptable salt thereof for the manufacture of medicaments for treating pancreatic cancer in patients identified or diagnosed as having pancreatic cancer.

In the various embodiments of this aspect of the present disclosure, the treatment method optionally also comprises a step of diagnosing or identifying a patient as having pancreatic cancer. The identified patient is then treated with or administered with a therapeutically effective amount of trans-[tetrachlorobis(1H-indazole)ruthenate(III)] (e.g., sodium trans-[tetrachlorobis(1H-indazole)ruthenate(III)] or indazolium trans-[tetrachlorobis(1H-indazole)ruthenate(III)]). Pancreatic cancer can be diagnosed in any conventional diagnostic methods known in the art including ultrasound, CT scan, MRI, Endoscopic ultrasound, CA19-9 (carbohydrate antigen 19.9) screening, and biopsy (e.g., percutaneous needle biopsy).

In addition, it has also been surprisingly discovered that the compound sodium trans-[tetrachlorobis(1H-indazole)ruthenate(III)] is equally effective in pancreatic cancer cell lines both sensitive and insensitive to drugs such as gemcitabine and erlotinib. Accordingly, provided herein is a method of treating refractory pancreatic cancer comprising treating a patient identified as having refractory pancreatic cancer with a therapeutically effective amount of trans-[tetrachlorobis(1H-indazole)ruthenate(III)] (e.g., sodium trans-[tetrachlorobis(1H-indazole)ruthenate(III)] or indazolium trans-[tetrachlorobis(1H-indazole)ruthenate(III)]). In specific embodiments, the patient has a pancreatic cancer that is refractory to a treatment comprising one or more drugs selected from the group consisting of gemcitabine, erlotinib, mitomycin C, platinum agents (e.g., cisplatin, carboplatin, oxaliplatin), docetaxel, 5-FU and capecitabine. That is, the present invention is also directed to the use of trans-[tetrachlorobis(1H-indazole)ruthenate(III)] or a pharmaceutically acceptable salt thereof (e.g., sodium trans-[tetrachlorobis(1H-indazole)ruthenate(III)] or indazolium trans-[tetrachlorobis(1H-indazole)ruthenate(III)]) for the manufacture of medicaments for treating refractory pancreatic cancer, e.g., a pancreatic cancer refractory to one or more drugs chosen from gemcitabine, erlotinib, docetaxel, mitomycin C, platinum agents (cisplatin, carboplatin, oxaliplatin), 5-FU and capecitabine.

The term "refractory pancreatic cancer," as used herein refers to pancreatic cancer that either fails to respond favorably to an anti-neoplastic treatment that does not include trans-[tetrachlorobis(1H-indazole)ruthenate(III)], or alternatively, recurs or relapses after responding favorably to an antineoplastic treatment that does not include trans-[tetrachlorobis(1H-indazole)ruthenate(III)]. Accordingly, "a pancreatic cancer refractory to a treatment" as used herein means a pancreatic cancer that fails to respond favorably to, or resistant to, the treatment, or alternatively, recurs or relapses after responding favorably to the treatment.

Thus, in some embodiments, in the method of the present disclosure, trans-[tetrachlorobis(1H-indazole)ruthenate(III)] or a pharmaceutically acceptable salt thereof is used to treat pancreatic cancer patients having a tumor that exhibits resistance to a treatment comprising one or more drugs selected from the group consisting of gemcitabine, erlotinib, mitomycin C, docetaxel, platinum agents (cisplatin, carboplatin, oxaliplatin), 5-FU and capecitabine. In other words, the method is used to treat a pancreatic cancer patient having previously been treated with a treatment regimen that includes one or more drugs selected from the group consisting of gemcitabine, erlotinib, mitomycin C, docetaxel, platinum agents (cisplatin, carboplatin, oxaliplatin), 5-FU and capecitabine, and whose pancreatic cancer was found to be non-responsive to the treatment regimen or have developed resistance to the treatment regimen. In other embodiments, the method is used to treat a pancreatic cancer patient previously treated with a treatment comprising one or more drugs selected from the group consisting of gemcitabine, erlotinib, mitomycin C, docetaxel, platinum agents (cisplatin, carboplatin, oxaliplatin), 5-FU and capecitabine, but the pancreatic cancer has recurred or relapsed, that is, a pancreatic cancer patient who has previously been treated with one or more such drugs, and whose cancer was initially responsive to the previously administered one or more such drugs, but was subsequently found to have relapsed. In specific embodiments, sodium trans-[tetrachlorobis(1H-indazole)ruthenate(III)] is used to treat pancreatic cancer patients previously treated with gemcitabine, i.e., who have a tumor that exhibits resistance to, or relapsed after a treatment including, gemcitabine. In other specific embodiments, sodium trans-[tetrachlorobis(1H-indazole)ruthenate(III)] is used to treat pancreatic cancer patients previously treated with erlotinib, i.e., who have a pancreatic cancer that exhibits resistance to, or relapsed after a treatment including, erlotinib. In yet other specific embodiments, sodium trans-[tetrachlorobis(1H-indazole)ruthenate(III)] is used to treat pancreatic cancer patients previously treated with a platinum cytotoxic agent (e.g., cisplatin, carboplatin, oxaliplatin, picoplatin), i.e., who have a pancreatic cancer that exhibits resistance to, or relapsed after a treatment including, a platinum cytotoxic agent (e.g., cisplatin, carboplatin, picoplatin, or oxaliplatin). In still other specific embodiments, sodium trans-[tetrachlorobis(1H-indazole)ruthenate(III)] is used to treat pancreatic cancer patients previously treated with 5-FU or capecitabine, i.e., who have a pancreatic cancer that exhibits resistance to, or relapsed after a treatment including, mitomycin C. In still other specific embodiments, sodium trans-[tetrachlorobis(1H-indazole)ruthenate(III)] is used to treat pancreatic cancer patients previously treated with mitomycin C, i.e., who have a pancreatic cancer that exhibits resistance to, or relapsed after a treatment including, mitomycin C.

To detect a refractory pancreatic cancer, patients undergoing initial treatment can be carefully monitored for signs of resistance, non-responsiveness or recurring pancreatic cancer. This can be accomplished by monitoring the patient's cancer's response to the initial treatment which, e.g., may includes one or more drugs selected from the group consisting of gemcitabine, erlotinib, mitomycin C, docetaxel, platinum agents (cisplatin, carboplatin, oxaliplatin), 5-FU and capecitabine. The response, lack of response, or relapse of the cancer to the initial treatment can be determined by any suitable method practiced in the art. For example, this can be accomplished by the assessment of tumor size and number. An increase in tumor size or, alternatively, tumor number, indicates that the tumor is not responding to the chemotherapy, or that a relapse has occurred. The determination can be done according to the "RECIST" criteria as described in detail in Therasse et al, J. Natl. Cancer Inst. 92:205-216 (2000).

In accordance with yet another aspect of the present disclosure, a method is provided for preventing or delaying the onset of pancreatic cancer, or preventing or delaying the recurrence of pancreatic cancer, which comprises treating a patient in need of the prevention or delay with a prophylatically effective amount of trans-[tetrachlorobis(1H-indazole)ruthenate(III)] or a pharmaceutically acceptable salt thereof (e.g., sodium trans-[tetrachlorobis(1H-indazole)ruthenate(III)] or indozolium trans-[tetrachlorobis(1H-indazole)ruthenate(III)]).

It is now known that people with chronic pancreatitis have an increased risk of developing pancreatic cancer. In addition, people having genetic syndromes are also predisposed to developing pancreatic cancer, including those who have autosomal recessive ataxia-telangiectasia and autosomal dominantly inherited mutations in the *BRCA2* gene or *PALB2* gene, Peutz-Jeghers syndrome due to mutations in the *STK11*, hereditary non-polyposis colon cancer (HNPCC), familial adenomatous polyposis (FAP), and the familial atypical multiple mole melanoma-pancreatic cancer syndrome (FAMMM-PC) due to mutations in the *CDKN2A* gene. These people can all be candidates for preventing or delaying the onset of pancreatic cancer using a prophylatically effective amount of, trans-[tetrachlorobis(1H-indazole)ruthenate(III)] or a pharmaceutically acceptable salt thereof (e.g., sodium trans-[tetrachlorobis(1H-indazole)ruthenate(III)] or indazolium trans-[tetrachlorobis(1H-indazole)ruthenate(III)]). In addition, patients with a family history of pancreatic cancer can also be identified for the application of the present method of preventing or delaying the onset of pancreatic cancer.

For purposes of preventing or delaying the recurrence of pancreatic cancer, pancreatic cancer patients who have been treated and are in remission or in a stable or progression free state may be treated with a prophylatically effective amount of trans-[tetrachlorobis(1H-indazole)ruthenate(III) or a pharmaceutically acceptable salt thereof (e.g., sodium trans-[tetrachlorobis(1H-indazole)ruthenate(III)] or indazolium trans-[tetrachlorobis(1H-indazole)ruthenate(III)]) to effectively prevent or delay the recurrence or relapse of pancreatic cancer.

In the present invention, pancreatic cancer refers to exocrine pancreatic cancer such as adenocarcinomas, adenosquamous carcinomas, signet ring cell carcinomas, hepatoid carcinomas, colloid carcinomas, undifferentiated carcinomas, and undifferentiated carcinomas with osteoclast-like giant cells.

As used herein, the phrase "treating ... with..." or a paraphrase thereof means administering a compound to the patient or causing the formation of a compound inside the body of the patient.

In accordance with the present invention, a therapeutically effective amount of trans-[tetrachlorobis(1H-indazole)ruthenate(III)] or a pharmaceutically acceptable salt thereof (e.g., sodium trans-[tetrachlorobis(1H-indazole)ruthenate(III)] or indazolium trans-[tetrachlorobis(1H-indazole)ruthenate(III)]) alone as a single agent, or alternatively in combination with one or more other anti-cancer agents is used for the treatment of pancreatic cancer.

Example of pharmaceutically acceptable salts include alkali metal salts (e.g., sodium or potassium salt), indazolium salts, etc. An alkali metal salt, preferably sodium salt of trans-[tetrachlorobis(1H-indazole)ruthenate(III)] (i.e., sodium trans-[tetrachlorobis(1H-indazole)ruthenate(III)] or potassium trans-[tetrachlorobis(1H-indazole)ruthenate(III)]) is particularly useful.

Alkali metal salts of trans-[tetrachlorobis(1H-indazole)ruthenate(III)] can be made in any methods known in the art. For example, PCT Publication No. WO/2008/154553 discloses an efficient method of making sodium trans-[tetrachlorobis(1H-indazole)ruthenate(III)]. U.S. Patent No. 7,338,946 discloses indazolium trans-[tetrachlorobis(1H-indazole)ruthenate(III)]) and a formulation containing the indazolium salt.

The pharmaceutical compounds such as a pharmaceutically acceptable salt of trans-[tetrachlorobis(1H-indazole)ruthenate(III)] (e.g., trans-[tetrachlorobis(1H-indazole)ruthenate(III)], or indazolium trans-[tetrachlorobis(1H-indazole)ruthenate(III)]) can be administered through intravenous injection or any other suitable means at an amount of from 0.1 mg to 1000 mg per kg of body weight of the patient based on total body weight. The active ingredients may be administered at once, or may be divided into a number of smaller doses to be administered at predetermined intervals of time, e.g., once daily or once every two days. It should be understood that the dosage ranges set forth above are exemplary only and are not intended to limit the scope of this invention. The therapeutically effective amount of the active compound can vary with factors including, but not limited to, the activity of the compound used, stability of the active compound in the patient's body, the severity of the conditions to be alleviated, the total weight of the patient treated, the route of administration, the ease of absorption, distribution, and excretion of the active compound by the body, the age and sensitivity of the patient to be treated, and the like, as will be apparent to a skilled artisan. The amount of administration can be adjusted as the various factors change over time.

In accordance with the present invention, it is provided a use of a compound having trans-[tetrachlorobis(1H-indazole)ruthenate(III)] or a pharmaceutically acceptable salt thereof (e.g., an alkali metal salt of trans-[tetrachlorobis(1H-indazole)ruthenate(III)] such as sodium trans-[tetrachlorobis(1H-indazole)ruthenate(III)] or potassium trans-[tetrachlorobis(1H-indazole)ruthenate(III)], or indazolium trans-[tetrachlorobis(1H-indazole)ruthenate(III)]) for the manufacture of a medicament useful for treating pancreatic cancer. The medicament can be, e.g., in an injectable form, e.g., suitable for intravenous, intradermal, or intramuscular administration. Injectable forms are generally known in the art, e.g., in buffered solution or suspension.

In accordance with another aspect of the present disclosure, a pharmaceutical kit is provided comprising in a container a unit dosage form of a compound containing trans-[tetrachlorobis(1H-indazole)ruthenate(III)], or a pharmaceutically acceptable salt thereof (e.g., an alkali metal salt of trans-[tetrachlorobis(1H-indazole)ruthenate(III)] such as sodium trans-[tetrachlorobis(1H-indazole)ruthenate(III)] or potassium trans-[tetrachlorobis(1H-indazole)ruthenate(III)], or indazolium trans-[tetrachlorobis(1H-indazole)ruthenate(III)]), and optionally instructions for using the kit in the methods in accordance with the present disclosure, e.g., treating, preventing or delaying the onset of pancreatic cancer, or preventing or delaying the recurrence of pancreatic cancer, or treating refractory pancreatic cancer. As will be apparent to a skilled artisan, the amount of a therapeutic compound in the unit dosage form is determined by the dosage to be used on a patient in the methods of the present disclosure. In the kit, a compound having trans-[tetrachlorobis(1H-indazole)ruthenate(III)] or a pharmaceutically acceptable salt thereof (e.g., an alkali metal salt of trans-[tetrachlorobis(1H-indazole)ruthenate(III)] such as sodium trans-[tetrachlorobis(1H-indazole)ruthenate(III)] or potassium trans-[tetrachlorobis(1H-indazole)ruthenate(III)], or indazolium trans-[tetrachlorobis(1H-indazole)ruthenate(III)]) can be in lyophilized form in an amount of, e.g., 25 mg, in an ampoule. In the clinic, the lyophilized form can be dissolved in a buffer and administered to a patient in need of the treatment in accordance with the present disclosure.

EXAMPLE

The compound sodium trans-[tetrachlorobis(1H-indazole)ruthenate(III)] was tested in a 3-dimentional tumor model derived from pancreatic tumor cell line MIA PaCa2. Specifically, cells were trypsinized, washed, counted by trypan blue exclusion. Tumor beads were then prepared by mixing 20,000 cells/10μl of HuBiogel (4 mg/mL) *(See* US Patent Application Serial No. 10/546,506.

The 3-D tumor beads were cultivated for 72 hours in multi-well plates with complete media (10% FBS) in a 37°C incubator +5% $CO_2$. Mini-tumors were treated with various concentrations of the test compound sodium trans-[tetrachlorobis(1H-indazole)ruthenate(III)] in media (final 0.2-0.3% DMSO) or control (DMSO). Repeated drug treatment was done by removing the culture media and replacing with fresh media with drug compound or DMSO. On Day 3, MTT assay and live-cell staining with Calcein AM were performed (5 beads/assay set).

Sodium trans-[tetrachlorobis(1H-indazole)ruthenate(III)] exhibited dose-dependent tumor killing effective in live-cell staining/image analysis, and significantly inhibited tumor proliferation activity. *See* Figure 1. Statistical analysis of data sets (Average, T-test, GI-50) was performed using MS-Excel program. The T-test result is shown in Table 1 below. The average GI-50 (the drug concentration required for growth inhibition at 50%) is 35.73 μM.

Table 1

| t-test | 200 | 100 | 50 |
|---|---|---|---|
| MIA-PaCa (control vs. experiment) | 5.71322E-09 | 6.01707E-10 | 3.35631E-08 |
| | control vs. 200μM | control vs. 100μM | control vs. 50μM |

EXAMPLE 2

To test the activities of sodium trans-[tetrachlorobis(1H-indazole)ruthenate(III)], ATCC's MTT Cell Proliferation Assay® was performed using human pancreatic cancer cell lines PANC-1 and Capan-1. Stock cultures were allowed to grow to 70-80% confluence for this study. The anti-proliferative activity of sodium trans-[tetrachlorobis(1H-indazole)ruthenate(III)], against the indicated cell lines was evaluated *in vitro* using the ATCC's MTT Cell Proliferation Assay (Catalog No. 30-1010K). PANC-1 cells were grown in DMEM medium with 10% FBS and 1% pen/strep/glutamine. Capan-1 cells were grown using IMDM medium 20% FBS and 1% of pen/strep/glutamine. Panc 1 and Capan-1 cell plates were seeded with 6000 cells/well and 15,000 cells/well, repectively, and treated with sodium trans-[tetrachlorobis(1H-indazole)ruthenate(III)] at 1,000 $\mu$M, or a series of 4x dilutions thereof (250 $\mu$M, 62.5 $\mu$M, etc.). 100$\mu$l of medium was removed from each well at 72 hours post-treatment and 10$\mu$l MTT reagent was added to each well. The plates were incubated plate at 37°C for 4 hours and then 100$\mu$l of detergent was added. The plates were left overnight at room temperature in the dark and was read on a plate reader using SoftMax® Pro (version 5.2, Molecular Devices).

The absorbance data was analyzed as follows: Absorbance values were converted to Percent of Control and plotted against test agent concentrations for $IC_{50}$ calculations using SoftMax® Pro (version 5.2, Molecular Devices). The plate blank signal average was subtracted from all wells prior to calculating the Percent of Control. Percent of Control values were calculated by dividing the absorbance values for each test well by the No Drug Control average (column 11 values; cells + vehicle control) and multiplying by 100. Plots of Compound Concentration versus Percent of Control were analyzed using the 4-parameter equation to obtain $IC_{50}$ values and other parameters that describe the sigmoidal dose response curve.

The $IC_{50}$ value for the test agents was estimated by curve-fitting the data using the following four parameter-logistic equation:

$$Y = \frac{Top - Bottom}{1 + \left(X / IC_{50}\right)^{n}} + Bottom$$

wherein "Top" is the maximal % of control absorbance (100%), "Bottom" is the minimal % of control absorbance at the highest agent concentration (down to zero), Y is the Percent of Control absorbance, X is the test agent Concentration, $IC_{50}$ is the concentration of agent that inhibits cell growth by 50% compared to the control cells, n is the slope of the curve. The $IC_{50}$ of sodium trans-[tetrachlorobis(1H-indazole)ruthenate(III)] in PANC-1 and Capan-1 cell lines were 30.6 $\mu$M and 45.2 $\mu$M, respectively.

Table 2

| Cell Line | $IC_{50}$ |
|-----------|-----------|
| Panc-1 | 30.6 $\mu$M |
| Capan-1 | 45.2 $\mu$M |

It is known that PANC-1 cells are resistant to both gemcitabine and erlotinib. *See* Guo et al., Tumori., 95:796-803 (2009); Durkin et al., Am. J. Surg., 186:431-436 (2003). Thus, the compound sodium trans-[tetrachlorobis(1H-indazole)ruthenate(III)] is active in cells resistant to gemcitabine and erlotinib.

All publications and patent applications mentioned in the specification are indicative of the level of those skilled in the art to which this invention pertains.

The mere mentioning of the publications and patent applications does not necessarily constitute an admission that they are prior art to the instant application.

**Claims**

1. Use of a therapeutically effective amount of a pharmaceutically acceptable salt of trans-[tetrachlorobis(1H-indazole)ruthenate(III)] for the manufacture of a medicament for treating pancreatic cancer.

2. Use of a therapeutically effective amount of a pharmaceutically acceptable salt of trans-[tetrachlorobis(1H-indazole)ruthenate(III)] for the manufacture of a medicament for preventing or delaying the onset of pancreatic cancer.

3. The use according to Claim 1, wherein said treatment is for preventing or delaying the onset of, a refractory pancreatic cancer.

4. The use according to Claim 3, wherein said cancer is refractory to a treatment comprising one or more of gemcitabine and erlotinib.

5. The use according to Claim 3, wherein said cancer was previously treated with and not responsive to a treatment comprising one or more of gemcitabine and erlotinib.

6. The use according to Claim 3, wherein said cancer is pancreatic cancer relapsing after a previous treatment comprising one or more of gemcitabine and erlotinib.

7. The use according to any one of Claims 4-6, wherein said previous treatment comprises gemcitabine.

8. The use according to any one of Claims 4-6, wherein said previous treatment comprises erlotinib.

9. The use according to any one of Claims 1-8, wherein said pharmaceutically acceptable salt is sodium trans-[tetrachlorobis(1H-indazole)ruthenate(III)].


**Patentansprüche**

1. Verwendung einer therapeutisch wirksamen Menge eines pharmazeutisch verträglichen trans-[tetrachlorobis(1H-indazol)ruthenat(III)]-Salzes zur Herstellung eines Medikaments zur Behandlung von Bauchspeicheldrüsenkrebs.

2. Verwendung einer therapeutisch wirksamen Menge eines pharmazeutisch verträglichen trans-[tetrachlorobis(1H-indazol)ruthenat(III)]-Salzes zur Herstellung eines Medikaments zur Vorbeugung oder Verzögerung des Ausbruchs von Bauchspeicheldrüsenkrebs.

3. Verwendung nach Anspruch 1, wobei die Behandlung zur Vorbeugung oder Verzögerung des Ausbruchs eines Bauchspeicheldrüsenkrebses ist, der nicht auf eine Therapie anspricht.

4. Verwendung nach Anspruch 3, wobei der Krebs nicht auf eine Therapie anspricht, die Gemcitabin und/oder Erlotinib umfasst.

5. Verwendung nach Anspruch 3, wobei der Krebs zuvor behandelt wurde mit einer Behandlung, die Gemcitabin und/oder Erlotinib umfasst und nicht angesprochen hat auf eine Behandlung, die Gemcitabin und/oder Erlotinib umfasst.

6. Verwendung nach Anspruch 3, wobei der Krebs Bauchspeicheldrüsenkrebs ist, der rezidiviert nach einer vorherigen Behandlung, die Gemcitabin und/oder Erlotinib umfasst.

7. Verwendung nach einem der Ansprüche 4 bis 6, wobei die vorherige Behandlung Gemcitabin umfasst.

8. Verwendung nach einem der Ansprüche 4 bis 6, wobei die vorherige Behandlung Erlotinib umfasst.

9. Verwendung nach einem der Ansprüche 1 bis 8, wobei das pharmazeutisch verträgliche Salz Natrium-trans-[tetrachlorobis(1H-indazol)ruthenat(III)] ist.


**Revendications**

1. Utilisation d'une quantité thérapeutiquement efficace d'un sel pharmaceutiquement acceptable de trans-[tétrachlorobis(1H-indazole)ruthénate (III)] pour la fabrication d'un médicament destiné à traiter le cancer pancréatique.

2. Utilisation d'une quantité thérapeutiquement efficace d'un sel pharmaceutiquement acceptable de trans-[tétrachlorobis(1H-indazole)ruthénate (III)] pour la fabrication d'un médicament destiné à prévenir ou retarder l'apparition du cancer pancréatique.

3. Utilisation selon la revendication 1, dans laquelle ledit traitement est destiné à prévenir ou retarder l'apparition d'un cancer pancréatique réfractaire.

**4.** Utilisation selon la revendication 3, dans laquelle ledit cancer est réfractaire à un traitement comprenant un ou plus de la gemcitabine et de l'erlotinib.

**5.** Utilisation selon la revendication 3, dans laquelle ledit cancer a été précédemment traité avec et ne répond pas à un traitement comprenant un ou plus de la gemcitabine et de l'erlotinib.

**6.** Utilisation selon la revendication 3, dans laquelle ledit cancer est un cancer pancréatique récurrent après un traitement précédent comprenant un ou plus de la gemcitabine et de l'erlotinib.

**7.** Utilisation selon l'une quelconque des revendications 4 à 6, dans laquelle ledit traitement précédent comprend la gemcitabine.

**8.** Utilisation selon l'une quelconque des revendications 4 à 6, dans laquelle ledit traitement précédent comprend l'erlotinib.

**9.** Utilisation selon l'une quelconque des revendications 1 à 8, dans laquelle ledit sel pharmaceutiquement acceptable est le trans-[tétrachlorobis(1H-indazole)ruthénate (III)] de sodium.

**Figure 1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008154553 A
- US 7338946 B
- US 546506 A

**Non-patent literature cited in the description**

- **RIVERA et al.** *Cancer Treatment Reviews,* 2009, 335-339
- **MOORE et al.** *J. Clin. Oncol.,* 2007, vol. 25 (15), 1960-6
- **KAPITZA et al.** *J. CancerRes. Clin. Oncol.,* 2005, vol. 131 (2), 101-10
- **THERASSE et al.** *J. Natl. Cancer Inst.,* 2000, vol. 92, 205-216
- **GUO et al.** *Tumori.,* 2009, vol. 95, 796-803
- **DURKIN et al.** *Am. J. Surg.,* 2003, vol. 186, 431-436